(19) Europäisches Patentamt
European Patent Office
Office européen des brevets

(11) **EP 2 434 998 B1**

(12) **EUROPÄISCHE PATENTSCHRIFT**

(45) Veröffentlichungstag und Bekanntmachung des Hinweises auf die Patenterteilung:
**04.05.2016   Patentblatt 2016/18**

(21) Anmeldenummer: **09776651.3**

(22) Anmeldetag: **26.05.2009**

(51) Int Cl.:
*A61F 9/01* (2006.01)        *A61F 9/008* (2006.01)
*B23K 26/08* (2006.01)

(86) Internationale Anmeldenummer:
**PCT/EP2009/003730**

(87) Internationale Veröffentlichungsnummer:
**WO 2010/136050 (02.12.2010 Gazette 2010/48)**

(54) **SYSTEM FÜR DIE LASERCHIRURGISCHE OPHTHALMOLOGIE**

SYSTEM FOR LASER SURGICAL OPHTHALMOLOGY

SYSTÈME D'OPHTALMOLOGIE PAR CHIRURGIE LASER

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(43) Veröffentlichungstag der Anmeldung:
**04.04.2012   Patentblatt 2012/14**

(73) Patentinhaber: **WaveLight GmbH**
**91058 Erlangen (DE)**

(72) Erfinder:
• **KITTELMANN, Olaf**
**14163 Berlin (DE)**

• **VOGLER, Klaus**
**99444 Blankenhain (DE)**

(74) Vertreter: **Katérle, Axel**
**Wuesthoff & Wuesthoff**
**Patentanwälte PartG mbB**
**Schweigerstraße 2**
**81541 München (DE)**

(56) Entgegenhaltungen:
**EP-A- 0 770 370        EP-A1- 1 977 725**
**WO-A-2005/058216        DE-A1-102005 014 760**
**US-A1- 2007 235 543        US-A1- 2008 212 623**

**Beschreibung**

Die Erfindung betrifft ein System für die laserchirurgische Ophthalmologie.

In der refraktiven ophthalmologischen Chirurgie werden durch Eingriffe am Auge eines Patienten die Brechungseigenschaften des Auges zur Korrektur von Sehfehlern verändert. Große Bedeutung hat dabei das sogenannte LASIK-Verfahren (LASer-Insitu-Keratomileusis), bei dem zunächst ein flächenhafter Hornhautschnitt gesetzt wird, durch den ein Deckelscheibchen - der sogenannte Flap - entsteht. Dieser kann zur Seite geklappt werden, um das darunter liegende Hornhautgewebe, das Stroma, freizulegen. Anschließend wird mit einem Laser (häufig einem Excimer-Laser) Stromagewebe nach Maßgabe eines patientenindividuell ermittelten Ablationsprofils ablatiert. Danach wird der Flap zurückgeklappt; die Wunde verheilt relativ schnell.

Zur Anbringung des Flapschnitts bei der LASIK wurde in jüngerer Zeit das früher verwendete mechanische Mikrokeratom durch einen Fs-Laser ersetzt, also einen Laser, der gepulste Laserstrahlung mit Pulsdauern im Femtosekundenbereich erzeugt. Für einen intrageweblichen Schnitt muss die Laserstrahlung im transmissiven Wellenlängenbereich der Kornea liegen, also über etwa 300 nm. Zugleich muss die Energiedichte im Strahlfokus groß genug sein, um einen optischen Durchbruch im Stroma zu erzeugen, die sogenannte Photodisruption. Deren Wirkbereich ist lokal auf den Fokusdurchmesser beschränkt. Um einen flächigen Schnitt zu erzeugen, muss deshalb der Strahlfokus nacheinander nach einem bestimmten Abtastmuster auf eine Vielzahl eng benachbarter, üblicherweise einander überlappender Punkte in der gewünschten Schnittfläche bzw. Schnittebene bewegt werden.

Die Vorteile einer Laserinzision gegenüber einem mechanisch mit einem Mikroskalpell gesetzten Hornhautschnitt führen zu einer zunehmenden Verbreitung des Einsatzes von Femtosekundenlasern bei LASIK-Operationen und anderen Behandlungen, bei denen Schnitte in die Hornhaut eingebracht werden sollen.

Bei der Durchführung eines Flapschnitts mittels eines Femtosekundenlasers wird in den meisten Fällen der Schnitt durch eine genau definierte Aneinanderreihung eng benachbarter Femtosekunden-Mikrodisruptionen erreicht. Dabei wird der Strahlfokus beispielsweise längs einer mäandrierenden, schlangenlinienförmigen Bahn in der Ebene des zu erzeugenden Flapschnitts geführt (sogenannter Linien-Scan). Dies schneidet das sogenannte Bett des Flaps. Anschließend wird ein finaler Randschnitt entlang des gewünschten Rands des Flaps gesetzt. Auf diese Weise wird der Flaprand definiert.

Die einzelnen Laserpulse werden in einer zur Strahlrichtung normalen Ebene (üblicherweise als x-y-Richtung bezeichnet) beispielsweise mittels eines Spiegel-Scanners präzise an der gewünschten Stelle in der Kornea positioniert. Alternativ zu einem Spiegelscanner kann beispielsweise ein Kristallscanner verwendet werden, um die gewünschte x-y-Ablenkung des Laserstrahls zu bewirken.

Die Qualität eines mit Fs-Laserstrahlung anzubringenden Schnitts wird von der präzisen Einhaltung von relevanten Parametern wie der Pulsenergie, dem Fokusdurchmesser, der Fokusebene sowie dem Abstand benachbarter Fokusorte (Spots) beeinflusst. Diese Parameter lassen sich getrennt für verschiedene Schnittführungen gut optimieren. Bei einem Flapschnitt beispielsweise kann zwischen zwei Formen von Schnittführung unterschieden werden, nämlich dem Flapbettschnitt, der das Bett des Flaps schneidet und dieses beispielsweise durch linienförmige, weitgehend parallel angeordnete Scanbahnen mit wechselnder Bewegungsrichtung abdeckt, und dem peripheren Randschnitt, der häufig notwendig ist für die Loslösung des Flaps vom Stroma.

Der Bahnverlauf einer Scanbahn, längs der der Laserstrahl bewegt wird, kann manchmal nicht optimal sein für die gewünschte Erzeugung einer athermischen (kalten) Photodisruption an jeder Stelle entlang der Scanbahn. Abhängig vom Bahnverlauf kann es zu lokalen Verdichtungen der Laserspots kommen. Beispielsweise kann bei einem mäandrierenden Linienscan, mit dem das Bett eines Flaps geschnitten werden soll, im Bereich der Umkehrbögen der einzelnen Liniensegmente eine Häufung der Spots pro Längen- oder Flächeneinheit im Vergleich zur Anzahl der Spots im Bereich der geradlinigen Bahnsegmente auftreten. Diese Häufung oder Verdichtung ist bedingt durch die Trägheit des Scanners, insbesondere bei Verwendung eines Spiegelscanners, an den Wendepunkten, wo sich die Scanrichtung umkehrt. Benachbarte Fokuspunkte sind dann möglicherweise nicht mehr klar voneinander separiert, sondern liegen so nahe beieinander, dass eine thermische Schädigung des Korneagewebes infolge zu hoher lokaler Energieeinstrahlung nicht mehr ausgeschlossen werden kann. Dennoch kann für das restliche Gebiet des Flaps, also das eigentliche Bett, das Schnittergebnis mit den gewählten Strahlparametern optimal sein.

Die Druckschrift EP 1 977 725 A1 beschreibt eine Vorrichtung für die refraktive Augenchirurgie mit einem gepulsten Femtosekundenlaser, bei der - etwa zur Erzeugung eines Flapschnitz der Kornea - zur Vermeidung einer regelmäßigen, unerwünschte Wölbungserscheinungen hervorrufenden Gitterstruktur einzelne Fokusse so positioniert werden, dass die Abstände benachbarter Fokusse großteils variieren. Die die Fokuspunkte derzeitlich sequenziellen Laserpulse werden in Zeilen geführt.

Die Druckschrift EP 0 770 370 A2 beschreibt eine Vorrichtung zur photospallativen Ablation von kornealem Gewebe und offenbart in diesem Zusammenhang, einen Laserstrahl auszutasten, wenn bei einer meandrierenden Scanbahn der Strahlfokus auf Bereiche außerhalb einer gewünschten Behandlungsregion fällt.

Die Druckschrift US 2008/0212623 A1 beschreibt eine Vorrichtung zur Präparation einer intrakornealen Lamelle mittels Laserstrahlung. Dabei wird sowohl ein anteriorer Schnitt als auch ein posteriorer Schnitt jeweils in zwei Teilen erzeugt, nämlich einem kreisförmigen Mittelteil und einem daran anschließenden ringförmigen Außenteil. In den Zentralbereichen kann hier mit geringerer Energie gearbeitet werden als in den Außenbereichen.

Die Druckschriften WO 2005/058216 A1 und DE 10 2005 014 760 A1 offenbaren Modulatoren, mit denen in Laserpulsfolgen selektiv einzelne Pulse abgeschwächt werden können.

Es ist somit eine Aufgabe der vorliegenden Erfindung, eine gerätetechnische Lösung zu schaffen, die es ermöglicht, bei der Anbringung von Inzisionen im Augengewebe mittels kurzpulsiger Laserstrahlung die Gefahr unerwünschter thermischer Schädigungen des Augengewebes zu verringern.

Zur Lösung dieser Aufgabe sieht die Erfindung ein System für die laserchirurgische Ophthalmologie gemäß Anspruch 1 vor. Die Erfindung geht somit von der Erkenntnis aus, dass es entlang der Scanbahn eines Laserstrahls Bereiche geben kann, in denen bedingt durch den Bahnverlauf ein erhöhter flächenbezogener Energieeintrag auftreten kann, bei ansonsten gleichbleibenden Strahlungsparametern. Der hieraus resultierenden Gefahr einer thermischen Schädigung begegnet die Erfindung, indem sie gezielt in vorbestimmten Bereichen der Scanbahn den flächenbezogenen Energieeintrag durch geeignete Austastung ausgewählter Laserpulse absenkt. Die Austastung wird auf einen Teil der Pulse in dem betreffenden Bahnbereich angewendet. Beispielsweise ist es möglich, nur jeden zweiten, jeden dritten oder allgemein jeden N-ten Puls in dem betreffenden Bahnbereich auszutasten. Austastung heißt, dass der betreffende Laserpuls vollständig geblockt oder geeignet abgelenkt und absorbiert wird, so dass im wesentlichen nichts von ihm auf das Augengewebe gelangt.

Gleichgültig, ob ausgewählte Pulse ausgetastet werden, bleiben entlang der gesamten Scanbahn solche Strahlungsparameter wie die Repetitionsrate der von der Quelle abgegebenen Laserpulse oder/und die Spotgröße (Fokusdurchmesser) vorzugsweise unverändert.

Die Steuerung der Modulatoreinheit durch die Steuereinheit erfolgt zweckmäßigerweise ortsabhängig, d.h. abhängig von dem Ort oder dem Bereich entlang der Scanbahn bzw. des Strahlablenkungsmusters, wo sich der Strahlfokus gerade befindet. Alternativ oder zusätzlich kann die Steuerung im Zusammenhang mit einer Geschwindigkeit der Laserstrahlung relativ zu dem Augengewebe, mit einer Veränderung der genannten Geschwindigkeit - also der Beschleunigung - oder mit einer Pulsenergie der Laserstrahlung stattfinden.

Auf diese Weise ist es möglich, in Abhängigkeit von Informationen bezüglich des Laserstrahlfokus die an das Augengewebe abgegebene Pulsenergie geeignet anzupassen. Dies kann, wie erwähnt, orts- bzw. positionsabhängig geschehen. Alternativ oder zusätzlich kann in Abhängigkeit von einem dem Strahlablenkungsmuster zugeordneten Bewegungsmuster - wie etwa einem Geschwindigkeitsprofil des Laserstrahlfokus - oder beispielsweise in Abhängigkeit von Informationen, die von der Scannereinheit oder anderen Systemkomponenten zur Verfügung gestellt werden, eine geeignete Modulation aufgeprägt werden.

Bei dem Schlangenlinien-Strahlablenkungsmuster, das aus einer Vielzahl nebeneinander im wesentlichen parallel verlaufender gerader Linienbahnen zusammengesetzt ist, erfolgt an den Randbereichen der Schnittgeometrie eine Umkehrung der Bewegungsrichtung um ca. 180°. An diesen Stellen des Strahlablenkungsmusters, die hier als Umkehrbögen bezeichnet sind, tritt aufgrund einer dem Scanner innewohnenden Trägheit eine Verlangsamung der Scangeschwindigkeit auf. Bei einer im wesentlichen gleichbleibenden Repetitionsrate der Laserquelle, d.h. bei einer im wesentlichen konstanten Pulsrate der Laserstrahlung, ergibt sich bei einer Reduzierung der Scangeschwindigkeit ein erhöhter Energieeintrag in das Augengewebe pro Flächeneinheit. Eine durch die Steuereinheit veranlasste Austastung einzelner Pulse oder ganzer Pulsfolgen im Bereich der Umkehrbögen kann einer aus dem erhöhten Energieeintrag möglicherweise resultierenden schädigenden thermischen Belastung entgegenwirken.

Eine Ausführungsform sieht vor, dass die Modulatoreinheit eine optische Gitterkomponente mit veränderlicher Beugungseffizienz umfasst. Die durch die Gitterkomponente hervorgerufene Beugung kann entweder den Laserstrahl vollständig austasten, indem sie ihn vollständig in eine gegebenenfalls vorhandene Strahlenfalle lenkt.

Bevorzugt umfasst die Modulatoreinheit einen akustooptischen oder einen elektrooptischen Modulator. Mit einem derartigen Modulator kann die Laserstrahlung sehr schnell und über ein definiertes kurzes Zeitintervall beispielsweise unterbrochen werden, um eine unerwünschte lokale Überlagerung mehrerer Laserstrahlungspulse an dem gleichen Ort zu vermeiden.

Die Erfindung wird nachfolgend anhand der beigefügten Zeichnungen näher erläutert. Es stellen dar:

Fig. 1 ein schematisches Ausführungsbeispiel eines erfindungsgemäßen Systems für die laserchirurgische Ophthalmologie,

Fig. 2 ein erstes beispielhaftes, erfindungsgemäßes Scanmuster für einen Flapschnitt,

Fig. 3 ein zweites beispielhaftes, nicht erfindungsgemäßes Scanmuster für einen Flapschnitt und

Fig. 4 ein drittes beispielhaftes, nicht erfindungsgemäßes Scanmuster für einen Flapschnitt.

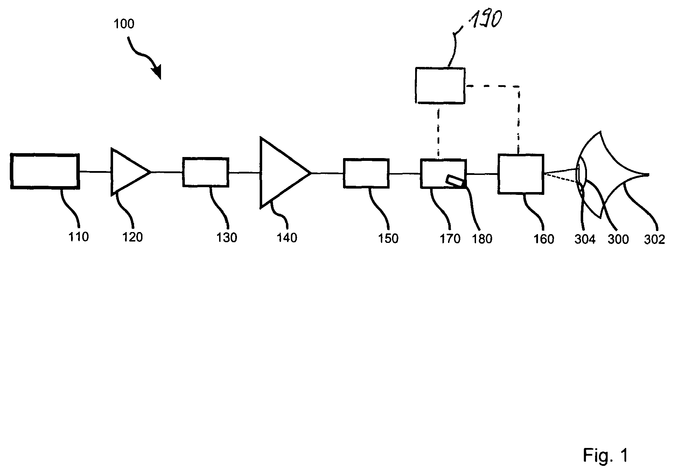

Das in Fig. 1 in schematischer Blockdarstellung gezeigte System, allgemein bezeichnet mit 100, ist ein für die Erzeugung einer intrageweblichen Inzision im Auge eines Patienten geeignetes Lasersystem. Ein intrakornealer Flapschnitt zur Erzeugung eines LASIK-Flaps ist ein mögliches und bevorzugtes Beispiel einer Inzision, für die das Lasersystem 100 geeignet ist. Es ist jedoch nicht ausgeschlossen, mit dem Lasersystem 100 andere Formen einer Gewebeinzision im Auge zu erzeugen.

Das Lasersystem 100 umfasst einen Laseroszillator 110, der freilaufend Laserpulse mit einer Dauer im Femtosekundenbereich und einer bestimmten Repetitionsrate emittiert. Der Laseroszillator 110 kann beispielsweise ein Festkörperlaseroszillator sein, insbesondere ein Faserlaseroszillator. Die von dem Laseroszillator 110 emittierten Pulse durchlaufen eine Vorverstärkeranordnung 120, welche die Pulsleistung der Pulse erhöht. Die Vorverstärkeranordnung 120 bewirkt gleichzeitig eine zeitliche Streckung der Pulse. Die so vorbehandelten Laserpulse werden sodann mittels eines sogenannten Pulse-Pickers 130 in ihrer Repetitionsrate reduziert. Der Laseroszillator 110 liefert beispielsweise Pulse mit einer Rate von 10 MHz oder größer. Diese Rate wird mit Hilfe des Puls-Pickers 130 auf beispielsweise 200 kHz reduziert. Die so in ihrer Repetitionsrate verringerten Pulse werden einem Leistungsverstärker 140 eingegeben, der die für die Applikation benötigte Pulsenergie der noch zeitlich gedehnten Pulse erzeugt. Bevor die so verstärkten Pulse einem Endpulskompressor 150 zugeführt werden, besitzen sie üblicherweise eine Pulslänge von über einer Pikosekunde, die von dem Endpulskompressor 150 wieder auf die durch die Bandbreite des Oszillators 110 und der Verstärkermedien ermöglichte kurze Fs-Pulsbreite von beispielsweise unter 500 fs komprimiert wird. Bei dem Endpulskompressor 150 kann es sich beispielsweise um einen Gitterkompressor handeln.

Die Komponenten 110, 120, 130, 140 und 150 kann man zusammengenommen als eine Laserquelle im Sinne der Erfindung betrachten.

Die so erzeugte Folge von Fs-Laserpulsen durchläuft anschließend einen Pulsmodulator 170, der beispielsweise als akustooptischer Modulator oder als elektrooptischer Modulator ausgebildet ist. Generell kann der Pulsmodulator 170 beliebige optisch wirksame Elemente enthalten, die ein schnelles Austasten bzw. Modulieren der Energie der Laserpulse ermöglichen. Ein akustooptischer Modulator kann beispielsweise Schaltzeiten von kleiner 10 μs bis hin zu beispielsweise 2 μs bei einer Auszeit von etwa 10 bis 100 μs bieten.

Dem Pulsmodulator 170 ist in Fig. 1 eine Strahlfalle (Beam Dump) 180 zugeordnet, welche dazu dient, etwaige auszutastende Pulse, die nicht auf das zu behandelnde Ziel gelangen sollen, zu absorbieren. Solche auszutastenden Pulse können von dem Pulsmodulator 170 auf die Strahlfalle 180 gelenkt werden, so dass sie im weiteren Strahlengang des auf das Ziel gerichteten Laserstrahls nicht mehr enthalten sind.

Nach dem Modulator 170 gelangt der Laserstrahl zu einer hier schematisch als ein gemeinsamer Block dargestellten Scan- und Fokussieranordnung 160, welche den Laserstrahl in einer zur Strahlrichtung senkrechten Ebene (x-y-Ebene) nach Maßgabe eines vorgegebenen Scan- bzw. Strahlablenkungsmusters ablenkt und in Strahlrichtung (z-Richtung) auf den gewünschten Zielort fokussiert. Der Zielort liegt im Fall einer Augenbehandlung im Augengewebe und insbesondere im Korneagewebe. Das Strahlablenkungsmuster definiert für die aufeinanderfolgen Laserpulse die Position jedes Pulses in der x-y-Ebene. Mit anderen Worten legt es eine Bahn (oder mehrere Bahnen) fest, längs welcher der Laserstrahl zu bewegen ist, um letztendlich die gewünschte Inzision zu erhalten.

Die Scan- und Fokussieranordnung 160 kann beispielsweise einen x-y-Spiegelscanner mit zwei galvanometrisch betriebenen, um zueinander senkrechte Achsen schwenkbaren Ablenkspiegeln für die Strahlabtastung und ein F-Theta-Objektiv zur Strahlfokussierung enthalten.

Die Pulsmodulator 170 und die Scan- und Fokussieranordnung 160 sind mit einer programmgesteuerten Steuereinheit 190 gekoppelt. Diese enthält in einem nicht näher dargestellten Programmspeicher ein Steuerprogramm, das bei Abarbeitung durch die Steuereinheit 190 eine solche Steuerung des Pulsmodulators 170 und der Scan- und Fokussieranordnung 160 bewirkt, dass der Laserstrahl in der gewünschten Zielebene fokussiert wird, entsprechend dem gewünschten Strahlablenkungsmuster über die Zielebene bewegt wird und in vorbestimmten, in dem Steuerprogramm definierten Teilen des Strahlablenkungsmusters mindestens ein Teil der Laserpulse durch den Pulsmodulator 170 energiemäßig abgeschwächt oder vollständig ausgetastet wird.

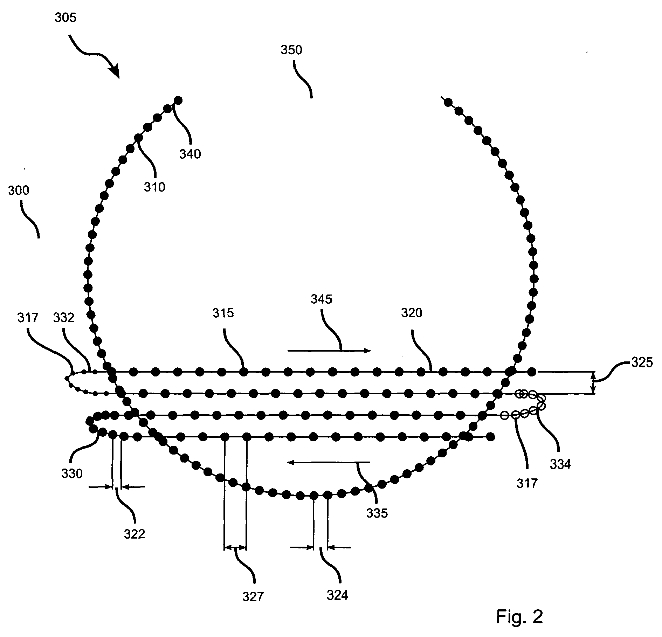

Der von der Scan- und Fokussieranordnung 160 ausgegebene Laserstrahl ist im gezeigten Beispielfall auf eine Kornea 300 eines humanen Auges 302 gerichtet und wird dort mit seinem Fokus in einer intrakornealen (planen oder nicht planen) Schnittebene 304 geführt. Diese Schnittebene 304 ist in vorliegenden stilisierten Schnittdarstellung des Auges 302 als Linie darstellt. Eine detaillierte Erläuterung der Schnittführung sowie der Funktionsweise des Modulators 170 im Zusammenhang mit der Schnittführung ergibt sich aus der folgenden Beschreibung der Fig. 2.

Fig. 2 zeigt einen Ausschnitt der menschlichen Kornea 300, an dem ein Flapschnitt gemäß einem ersten, erfindungsgemäßen Flapschnittschema 305 durchgeführt werden soll. Das Flapschnittschema 305 ist nur schematisch dargestellt, insbesondere entsprechen die Größenverhältnisse unter Umständen nicht den realen Verhältnissen. Zudem ist das Flapschnittschema 305 nur auschnittsweise angedeutet, um die Darstellung im Ganzen übersichtlich zu halten.

Zur Durchführung des Flapschnitts werden Laserpulse an den mit Kreisen veranschaulichten Stellen 310, 315 der Kornea 300 fokussiert, sodass Mikrodisruptionen entstehen. Die von dem System 100 erzeugte Laserstrahlung wird mittels eines Hochgeschwindigkeitsscanners über die Oberfläche der Kornea 300 geführt. Die Kornea weist in der Regel eine in erster Näherung als sphärisch zu bezeichnende Oberflächenkrümmung auf. Für die Durchführung eines Flapschnitts ist es beispielsweise üblich, die Oberfläche der zu behandelnden Kornea durch Anpressen/-saugen eines Aufsatzes zu applanieren. Die Fokussierung der Femtosekunden-Laserstrahlung erfolgt innerhalb einer Ebene 304 (siehe Fig. 1), die im wesentlichen senkrecht zur Sehachse des Auges verläuft, sodass eine im Wesentlichen gleichförmige Flapdicke entsteht. Der Laserstrahl wird innerhalb dieser Ebene entlang bestimmter Bahnkurven geführt.

In einem ersten Teil des Schnittschemas 305 wird der flächenförmige Flapbettschnitt erzeugt. Dazu wird der Laserstrahl entlang einer im Wesentlichen geraden Scanbahn 320 in einer ersten Bewegungsrichtung 335 geführt und wechselt seine Bewegungsrichtung bei Überschreiten des gewünschten Flapschnittradius in eine zweite Bewegungsrichtung 345 und wird anschließend wieder entlang einer Geraden parallel und mit einem bestimmten Abstand 325 zur ersten Scangeraden 320 geführt, sodass die gesamte Fläche des Flapschnitts raster- bzw. mäanderförmig mit wechselnden Bewegungsrichtungen 335, 345 abgetastet wird.

Innerhalb der einzelnen Scanlinien 320 reihen sich die Fokusorte 315 nahezu äquidistant mit einem Abstand 327 aneinander, da Pulsrate und Scangeschwindigkeit entlang der Linien 320 konstant gehalten werden. Die einzelnen Scanlinien 320 sind so mit einem Abstand 325 zueinander versehen, dass sich zusammen mit dem Abstand 327 der einzelnen Fokusorte 315 innerhalb der Scanlinie in der Gesamtheit ein flächenhafter Schnitt ergibt. An den Rändern innerhalb der Umkehrbögen 330 des Flapschnittmusters ändert sich die Bewegungsrichtung des Laserstrahls beispielsweise um etwa 180°. An diesen Umkehrbögen 330 ergibt sich aufgrund der Trägheit des Scanners eine verlangsamte Relativgeschwindigkeit zwischen Laserstrahl und Korneaoberfläche, sodass viele der Fokusorte 315 örtlich nahe beieinander liegen oder zusammenfallen. Dies zeigt sich in einem deutlich geringeren Fokusortabstand 322 innerhalb der Umkehrbögen 330 verglichen mit dem Fokusortabstand 327 entlang der Scanstreckenabschnitte 320. Diese Bereiche 330 unterliegen folglich einer potentiellen thermischen Schädigung.

Zur Vervollständigung des Flapschnitts wird nach dem mit den Linien 320 dargestellten Flächenschnitt ein Randschnitt entlang einer beispielsweise im Wesentlichen kreisförmigen Bahn 340 durchgeführt. Für den Randschnitt kann eine andere Fokusdichte erforderlich oder vorteilhaft sein verglichen beispielsweise mit der des Flapbettschnitts. Entsprechend ist der Abstand 324 der Fokusorte 310 entlang der Randschnittbahnkurve 340 im Ausführungsbeispiel der Fig. 2 geringer als der Abstand 327 der Fokusorte entlang der im Wesentlichen linearen Bahnkurven 320. Der Randschnitt 340 ist an einer Stelle 350 unterbrochen, die bei dem Ablösen und Aufklappen des abgetrennten Korneabereichs als Flapscharnier, das auch als Hinge bezeichnet wird, dient. Bei dem Aufklappen werden die potentiell thermisch geschädigten Bereiche 330 entlang der Linie 340 abgetrennt und liegen dann außerhalb des Flaps.

Eine erste erfindungsgemäße Möglichkeit, um die angesprochene thermische Schädigung an den Umkehrpunkten 330 zu verringern, besteht darin, die Abgabe der Laserstrahlung an die Kornea durch eine geeignete Ansteuerung des akustooptischen Modulators 170 zu unterbrechen, wenn die Fokusorte außerhalb der (zunächst gedachten) Randschnittlinie 340 fallen.

Diese Situation ist bei dem Umkehrbogen 334 dargestellt. Diejenigen Fokusorte 315 bzw. die damit verbundenen Mikrodisruptionen, die im Bereich 334 auf die Kornea 300 fallen bzw. ausgelöst würden, sind als nicht ausgefüllt Kreise dargestellt. Genauer wird in diesem Ausführungsbeispiel der Laserstrahlengang durch den Modulator 170 in einem Bereich außerhalb des Flaprands blockiert, sodass nur einzelne Pulse auf die Kornea 300 auftreffen. Dieses Austasten von Laserpulsen kann beispielsweise in Abhängigkeit von einem von der Scannereinheit 160 zur Verfügung gestelltem Orts-, Geschwindigkeit- oder Beschleunigungssignal erfolgen. Gegebenenfalls kann die Signalerzeugung und/oder -bereitstellung aber auch durch andere, von der Scannereinheit unabhängige Module oder Komponenten erfolgen. Ferner kann das Austasten gegebenenfalls auch durch eine rein zeitliche Steuerung bzw. Programmierung der Laserstrahlführung oder unter Berücksichtigung anderer geeigneter Signale erfolgen. Durch diese Maßnahme wird, wie in Fig. 2 zu erkennen ist, der Randbereich 334 zum Teil freigehalten von durch den Laserstrahl induzierten Mikrodisruptionen und eine thermische Schädigung in diesem Bereich ist ausgeschlossen.

Eine alternativ oder gegebenenfalls in Kombination mit der oben dargestellten Möglichkeit einsetzbare, nicht erfindungsgemäße Strategie zur Vermeidung einer thermischen Schädigung besteht in einer Modulation der Energie einzelner Femtosekundenpulse bei der Schnittführung in der Kornea. Dies ist in dem Umkehrbereich 332 der Fig. 2 dargestellt. Anstatt wie im Bereich 334 die Ortsdichte der einzelnen Fokusorte im statistischen Mittel im Wesentlichen annähernd innerhalb eines gewünschten Bereichs zu halten, wird im Bereich 332 die Energie verringert, die durch die einzelnen Laserpulse in Gestalt der Fokusorte 317 als Mikrodisruptionen an die Kornea abgegeben wird. Zur Darstellung sind die Kreise, die die Fokusorte der Laserstrahlung repräsentieren, als Kreise 317 mit kleinerem Radius dargestellt. Zur Erreichung einer geringeren Energieabgabe wird der akustooptische Modulator 170 nicht von einem On-Zustand in den absoluten Off-Zustand geschaltet. Vielmehr sind für prinzipiell jeden Puls der Femtosekundenpulsfolge individuelle Pulsenergien einstellbar, die auf die konkrete Anwendung in Größe und Abfolge angepasst werden können. Dabei sind Schaltzeiten realisierbar, die Einzelimpulse bei einer Repetitionsrate bis ca. 1 MHz modulieren können. Vorliegend wird für Pulse, die außerhalb des Flapschnittbereichs liegen, eine konstant niedrigere Pulsenergie eingestellt bzw. - geregelt. Es ist aber auch ein dem vermutlichen oder tatsächlichen Geschwindigkeits- bzw. Beschleunigungsverlauf angepasster Pulsenergieverlauf denkbar. Weiterhin ist es denkbar, den Umkehrbogen 332 nicht außerhalb des Flapbettes, sondern innerhalb des Randschnittes anzuordnen und so eine zeitliche Verkürzung des gesamten Flapschnittvorgangs durch einen Verzicht eines Scannens über den eigentlichen Randschnittbereich hinaus zu erreichen. Mit dem in Fig. 2 dargestellten Flapschnittschema 305 lassen sich beliebige Flapformen realisieren, was insbesondere bei Aberrationen höherer Ordnung der Korneageometrie wie etwa Astigmatismus von Vorteil sein kann.

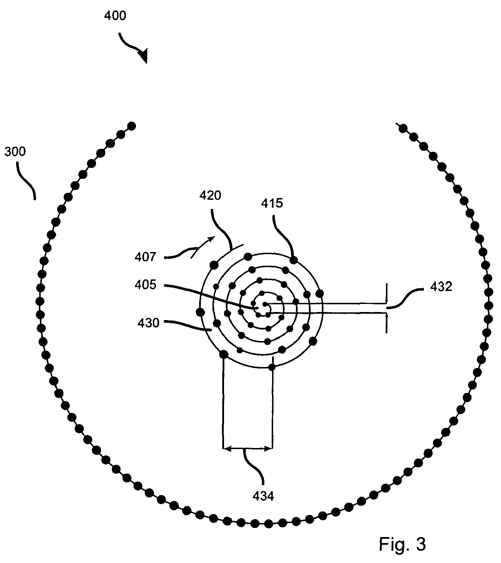

Eine weitere alternative, nicht erfindungsgemäße Form der Erzeugung eines Flapschnitts ist in Fig. 3 dargestellt. Anstelle eines linearen, mäanderförmigen Abscannens des Flapschnittbereichs ist bei dem Flapschnittschema 400 der Fig. 3 eine spiralförmige Scanführung vorgesehen. Die Darstellung des Schnittschemas ist wiederum nur schematisch, d.h. die Größen- und Abstandsverhältnisse sind wie in Fig. 2 nicht maßstabsgetreu und können in der Realität von dem dargestellten Schema abweichen. Ferner ist auch wie in Fig. 2 die Schnittführung unvollständig. Insbesondere im peripheren Bereich des Spiralschnitts sind bei einer realen Schnittführung noch weitere Pulse zu setzen.

Die Schnittführung erfolgt im vorliegenden Ausführungsbeispiel entlang einer sich vom Zentralbereich 405 der Kornea 300 nach außen zu peripheren Bereichen 430 entwickelnden Spiralbahn 420, vorliegend im Uhrzeigersinn entlang der mit einem Pfeil 407 in Fig. 3 angedeuteten Bewegungsrichtung. Die einzelnen Fokusorte 415 werden entlang der spiralförmigen Bahn 420 mit kontinuierlicher Pulsrate gesetzt. Das durch den Scanner erzeugte Geschwindigkeitsprofil entlang der Spiralbahn 420 setzt sich aus einer linear-radialen sowie einer Rotations-Geschwindigkeitskomponente zusammen. Bei einer konstanten Rotationskomponente (d.h. konstanter Winkelgeschwindigkeit) und einer konstanten radialen Komponente herrscht bei einer konstanten Pulsrate im Zentralbereich 405 eine deutlich höhere Fokusortdichte entlang der Bahnkurve 420 als in peripheren Bereichen 430, da im peripheren Bereich 430 aufgrund der konstanten Rotationsgeschwindigkeit die Bahngeschwindigkeit höher ist. Dies zeigt sich in einem kleineren Fokusortabstand 432 im Zentralbereich 405 verglichen mit dem Fokusortabstand 434 im peripheren Bereich 430.

Zwar hat das Flapschnittschema 400 den Vorteil, dass bei der beschriebenen Bewegungsrichtung 407 vom Zentrum 405 zu den peripheren Bereichen 430 der flächige Flapbettschnitt kontinuierlich in den Flaprandschnitt überführt werden kann. Es besteht aber andererseits die Gefahr einer thermischen Schädigung im Zentralbereich der Kornea 300, was dort von besonderem Nachteil sein kann. Auch bei einer Entwicklung der Spiralbahn in umgekehrter Bewegungsrichtung, d.h. von dem peripheren Randbereich 430 des Flaps nach innen in den Zentralbereich 405 besteht die selbe Gefahr, da auch hier eine Mischpulsleistung verwendet werden muss, die für den peripheren Bereich 430 bei zeitlich fester Pulsfrequenz tendenziell zu niedrig und im Zentralbereich 405 der Kornea 300 eventuell zu hoch ist.

Um eine gleichförmigeren Energieeintrag pro Flächeneinheit zu erzielen, wird gemäß einer Ausführungsform die an das Augengewebe der Kornea 300 an den Fokusorten 415 abgegebene Energie derart moduliert, dass in dem Zentralbereich 405 der Kornea der Energieeintrag geringer ist als in dem peripheren Bereich 430. Dies ist in Fig. 3 durch einen sich vom Zentralbereich 405 zu dem peripheren Bereich 430 vergrößernden Radius der die Fokusorte 415 darstellenden Kreise angedeutet. Somit reduziert sich zwar von innen 405 nach außen 430 die Fokusortdichte, durch die sich erhöhende Pulsleistung ist der durch die ausgelösten Mikrodisruptionen bewirkte Energieeintrag pro Fokusort höher und kompensiert somit die sich reduzierende Fokusortdichte zu einem in Wesentlichen innerhalb eines gewünschten Bereichs bleibenden Energieeintrag pro Flächeneinheit. Diese Kompensation mittels des Modulators 170 kann durch eine Steuereinheit gemäß einer vorher festgelegten mathematischen Funktion zeitlich gesteuert werden, es kann aber auch ein Regelkreis eingerichtet werden, der die Pulsleistung beispielsweise in Abhängigkeit von der radialen Position der Scaneinrichtung 160 regelt.

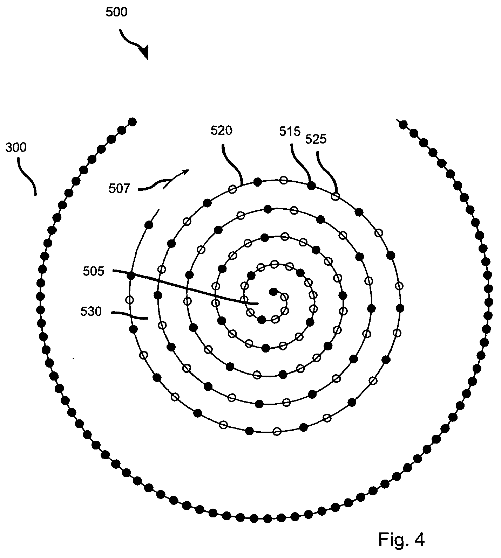

Alternativ zu einer Steuerung bzw. Regelung der Pulsleistung entlang der Spiralbahnkurve 420 kann bei einem Spiralbahnscanschema eine konstante Pulsdichte entlang einer Bahnkurve durch Austasten von Laserpulsen eingesteuert bzw. -geregelt werden. Dies ist schematisch in Fig. 4 dargestellt. Zur Vermeidung von Wiederholungen wird bei der Beschreibung der Figur 4 nur auf die wesentlichen Unterschiede zu den bereits beschriebenen Ausführungsformen der Fign. 2 und 3 eingegangen. In Fig. 4 ist ein dem Schema der Ausführungsform der Fig. 3 vergleichbares, nicht erfindungsgemäßes Flapschnittschema 500 dargestellt. Dieses erzeugt mittels eines spiralförmigen Strahlablenkungsmusters entlang einer Bahnkurve 520 einen Flapbettschnitt durch Aufbringen von Laserpulsen 515. Um den Fokusabstand der Laserpulse 515 in der Bahnkurve 520 des Spiralscans im Wesentlichen konstant zu halten, wird (anstatt einer Variation der Pulswiederholfrequenz der Laserquelle oder anstelle einer Variation der Laserpulsenergie) durch Austastung einzelner Laserpulse 525 die Pulsrate der auf das Augengewebe auftreffenden Laserstrahlung kontinuierlich geändert gemäß folgender Gleichung

$$s_f = const \sim \frac{d_i}{f_i} \cdots \frac{d_a}{f_a} \quad f_i << f_a \quad \text{da} \quad d_i << d_a$$

wobei

$f_i$ = Pulsrate im inneren Spiralbereich;
$f_a$ = Pulsrate im äußeren Spiralbereich und

$s_f$ = Spotabstand in der Bahnkurve.

$d_i$ = Durchmesser der Bahnkurve im zentralen Bereich;

$d_a$ = Durchmesser der Bahnkurve in einem äußeren Bereich.

Somit ergibt sich in einem zentralen Bereich 505 durch eine Austastung von drei von vier Pulsen und in einem peripheren Bereich 530 durch Austasten jedes zweiten Pulses eine annähernd gleichförmige Fokusortdichte über den gesamten Flapbettschnittbereich der Kornea 300. Die hier dargestellten Zahlenwerte und Größenverhältnisse sind unter Umständen nicht realitäts- bzw. maßstabsgetreu und dienen nur der schematischen Darstellung. In einer konkreten Ausführungsform können die wirklichen Puls-zu-Austastung-Verhältnisse von den vereinfacht dargestellten Werten erheblich abweichen.

Insgesamt kann somit die mit negativen Folgen verbundene lokale Häufung oder sogar Überlagerung mehrerer Fs-Laserpulse im Bereich der Umkehrpunkte bei einem linearen rasterförmigen Flapschnittverfahren oder eine zu dichte Abfolge von Fs-Laserpulsen bei einem spiralförmigen Scanverfahren durch ein programmkorreliertes Austasten oder durch eine gezielte Modulation der Pulsleistung der Laserstrahlung vermieden werden. Die Laserquelle läuft in allen Fällen ungestört mit festen und optimierten Strahlparametern wie Pulsenergie, Pulsdauer sowie Divergenz und Strahlparameterprodukt weiter, wodurch die Schnittqualität gleichmäßig optimiert bleibt.

Die Erfindung kann auch für andere Fs-Laseranwendungen in der Ophthalmologie genutzt werden. Beispielsweise können ähnliche Schnittschemata für die lamellare und penetrierende Keratoplastik wie etwa bei einer Lentikelextraktion o. ä. eingesetzt werden.

**Patentansprüche**

1. System (100) für die laserchirurgische Ophthalmologie, umfassend

   - eine Quelle (110) gepulster Laserstrahlung mit Strahlungsparametern, welche auf die photodisruptive Erzeugung einer Inzision in der Kornea abgestimmt sind,
   - einen Scanner (160) zur Ablenkung der Laserstrahlung,
   - eine elektronische Steuereinheit (190), welche dazu eingerichtet ist, den Scanner nach Maßgabe einer vorgegebenen, zur Erzeugung eines kornealen Flaps ausgelegten Schnittgeometrie zu steuern, wobei die Schnittgeometrie einen das Bett des Flaps definierenden Bettschnitt sowie einen den Rand des Flaps definierenden Randschnitt umfasst,

   **dadurch gekennzeichnet, dass** für den Bettschnitt ein Schlangenlinien-Strahlablenkungsmuster mit einer Vielzahl geradlinig nebeneinander verlaufender Linienbahnen (320) und einer Vielzahl jeweils ein Paar benachbarter Linienbahnen endseitig verbindender Umkehrbögen (334) festgelegt ist, wobei die Umkehrbögen außerhalb des Flaprands liegen, und dass das System eine Modulatoreinheit (170) zur Modulation der von der Quelle (110) abgegebenen Laserpulse umfasst, wobei die Steuereinheit (190) ferner dazu eingerichtet ist, die Modulatoreinheit (170) derart zu steuern, dass in außerhalb des Flaprands liegenden Teilen des Schlangenlinien-Strahlablenkungsmusters ein Teil der Laserpulse unterdrückt ist.

2. System nach Anspruch 1, wobei die Modulatoreinheit (170) einen akustooptischen oder einen elektrooptischen Modulator umfasst.

3. System nach einem der vorhergehenden Ansprüche, wobei die Modulatoreinheit (170) eine optische Gitterkomponente mit veränderlicher Beugungseffizienz umfasst.

4. System nach einem der vorhergehenden Ansprüche, wobei der Modulatoreinheit (170) eine Strahlfalle (180) zugeordnet ist.

**Claims**

1. System (100) for ophthalmic laser surgery, comprising

   - a source (110) of pulsed laser radiation with radiation parameters matched to the photodisruptive generation of an incision in the cornea,

- a scanner (160) for deflecting the laser radiation,
- an electronic control unit (190) which has been set up to control the scanner in accordance with a predetermined incision geometry designed for production of a corneal flap, wherein the incision geometry comprises a bed incision defining the bed of the flap as well as a marginal incision defining the edge of the flap,

**characterized in that** a serpentine beam-deflection pattern with a plurality of line paths (320) extending rectilinearly side by side and with a plurality of reversing bends (334) each terminally connecting a pair of adjacent line paths is established for the bed incision, wherein the reversing bends lie outside the edge of the flap, and that the system comprises a modulator unit (170) for modulating the laser pulses emitted from the source (110), the control unit (190) further being set up to control the modulator unit (170) in such a manner that in parts of the serpentine beam-deflection pattern lying outside the edge of the flap some of the laser pulses are suppressed.

2. System according to claim 1, wherein the modulator unit (170) includes an acousto-optical or an electro-optical modulator.

3. System according to any one of the preceding claims, wherein the modulator unit (170) includes an optical grating component with variable diffraction efficiency.

4. System according to any one of the preceding claims, wherein a beam dump (180) is assigned to the modulator unit (170).

**Revendications**

1. Système (100) d'ophtalmologie par chirurgie laser, comprenant

- une source (110) de rayonnement laser pulsé dont les paramètres de rayonnement sont ajustés pour réaliser par photodisruption une incision dans la cornée,
- un scanner (160) pour dévier le faisceau laser,
- une unité de commande électronique (190) qui est conçue pour commander le scanner en fonction d'une géométrie d'incision prédéfinie pour découper un volet cornéen, ladite géométrie d'incision prévoyant une découpe définissant le lit du volet et une découpe définissant le bord du volet,

**caractérisé en ce qu'**il est défini, pour la découpe définissant ledit lit, un modèle de déviation du faisceau en ligne sinueuse avec plusieurs lignes (320) rectilignes adjacentes et de nombreuses parties courbes de retour (334) reliant chacune une paire de lignes à leur extrémité, ces parties courbes de retour se trouvant en dehors du bord du volet cornéen, et **en ce que** le système comprend une unité de modulation (170) qui module les impulsions laser délivrées par la source (110), ladite unité de commande (190) étant en outre conçue pour commander l'unité de modulation (170) de telle manière qu'une partie des impulsions laser est supprimée dans les zones du modèle de déviation du faisceau en ligne sinueuse qui se trouvent en dehors du bord du volet cornéen.

2. Système selon la revendication 1, l'unité de modulation (170) comprenant un modulateur acousto-optique ou un modulateur électro-optique.

3. Système selon l'une des revendications précédentes, l'unité de modulation (170) comprenant un composant de réseau optique à efficacité diffractionnelle variable.

4. Système selon l'une des revendications précédentes, un piège de faisceau (180) étant associé à l'unité de modulation (170).

Fig. 1

Fig. 2

Fig. 3

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1977725 A1
- EP 0770370 A2
- US 20080212623 A1
- WO 2005058216 A1
- DE 102005014760 A1